# EUROPÄISCHE PATENTSCHRIFT

(11) **EP 1 430 361 B1**
(45) Veröffentlichungstag und Bekanntmachung des Hinweises auf die Patenterteilung: **06.07.2005**
(21) Anmeldenummer: 02769900.8
(22) Anmeldetag: 29.08.2002
(51) Int. Cl.: G03F 7/40, A61B 5/04, A61B 5/07, A61L 27/34, A61L 31/10, G03F 7/09

(54) **CARL FÜR BIOELEKTRONIK: SUBSTRATANBINDUNG ÜBER LEITFÄHIGE SCHICHT**
CARL FOR BIOELECTRONICS: SUBSTRATE LINKAGE USING A CONDUCTIVE LAYER
CARL EN BIOELECTRONIQUE : LIAISON A UN SUBSTRAT AU MOYEN D'UNE COUCHE CONDUCTRICE

(30) Priorität: 28.09.2001 DE 10147954
(43) Veröffentlichungstag der Anmeldung: 23.06.2004
(73) Patentinhaber: Infineon Technologies AG, 81669 München (DE)
(72) Erfinder: ELIAN, Klaus, 91056 Erlangen (DE)
(74) Vertreter: Müller - Hoffmann & Partner
(86) Internationale Anmeldenummer: PCT/DE2002/003167
(87) Internationale Veröffentlichungsnummer: WO 2003/032086

(56) Entgegenhaltungen:
- EP-A- 0 442 674
- DE-A- 10 133 256
- US-A- 5 561 030
- CUI X ET AL: "Electrochemical deposition and characterization of conducting polymer polypyrrole/PSS on multichannel neural probes" SENSORS AND ACTUATORS A, ELSEVIER SEQUOIA S.A., LAUSANNE, CH, Bd. 93, Nr. 1, 25. August 2001 (2001-08-25), Seiten 8-18, XP004255500 ISSN: 0924-4247
- CUI, XINYAN ET AL: "Surface Modification of Neural Recording Electrodes with Conducting Polymer/Biomolecule Blends" JOURNAL OF BIOMEDICAL MATERIALS RESEARCH, Bd. 56, Nr. 2, 30. April 2001 (2001-04-30), Seiten 261-272, XP008015603 WILEY, NEW YORK, NY, US ISSN: 0021-9304

## Beschreibung

Die Erfindung betrifft ein Verfahren zur Herstellung biokompatibler Strukturen sowie einen biokompatiblen Mikrochip.

Die Bioelektronik ist ein sich schnell entwickelndes Forschungsgebiet, das Chemie, Biochemie und Physik verbindet. Ihr Ziel ist die Kommunikation zwischen elektronischen Vorrichtungen und lebenden Zellen. Hauptmerkmal eines bioelektronischen Bauteils ist die Immobilisierung eines Biomaterials auf einem leitenden oder halbleitenden Träger und die Umwandlung von biologischen Funktionen, die mit dem biologischen Material verbunden sind, in elektronische Signale. Beispiele für mikroelektronische Bauteile, mit denen biologische Funktionen beeinflusst und gesteuert werden können, sind Herzschrittmacher sowie Innenohr-Hörprothesen. Die Entwicklung derartiger bioelektronischer Bauteile führt hin zu immer komplexeren Systemen, in denen eine Vielzahl von Übertragungskanälen für den Informationsübergang zwischen elektronischem Bauteil und den zu beeinflussenden Zellen erforderlich sind. So befinden sich zum Beispiel Retina-Implantate oder Geh-/Stehprothesen in der Entwicklung. Dafür ist es notwendig, Implantate zu entwickeln, die mit zahlreichen Kontaktpunkten sowohl Nervengewebe in zeitlich versetzter Abfolge stimulieren als auch eine Vielzahl von Nervensignalen räumlich und zeitlich gut aufgelöst erfassen können. Hierbei scheiden allerdings metallische Elektroden, wie sie bei Herzschrittmachern verwendet werden, meist aus, da diese als Fremdkörper erkannt werden und so zu Abstoßungsreaktionen führen. Man versucht daher, den elektrischen Kontakt zwischen elektronischem Bauteil und biologischem Gewebe mit Hilfe von Polymeren, wie zum Beispiel Silikonen oder Polyurethan herzustellen. Dazu müssen die Polymere allerdings elektrisch leitend und zusätzlich biokompatibel sein, das heißt, die Materialien dürfen keine Abstoßungsreaktion auslösen. Um Nervenbahnen gezielt ankontaktieren zu können, ist eine Strukturierung dieser Materialien oder der verwendeten Substrate, zum Beispiel Minisiliziumplättchen mit Abmessungen im Bereich von wenigen mm, notwendig. Die Größe der in bzw. auf dem Substrat erzeugten Strukturen, wie Pyramiden oder Löcher, liegt dabei in einem Bereich von 10 µm bis ungefähr 70 µm. Das kritischste Element in der Bioelektronik ist die Schnittstelle zwischen Elektronik und biologischem Gewebe. Um einen geeigneten Kontakt herzustellen, geht man bisher zum Beispiel in der Weise vor, dass zunächst ca. 25 µm tiefe pyramidenförmige Vertiefungen in einen Siliziumchip eingeätzt werden. Die Vertiefungen werden anschließend zunächst mit leitfähigem Silikon angefüllt und anschließend eine zweite Schicht aus nicht leitfähigem Silikon aufgetragen. Die Polymeren werden anschließend vernetzt und die strukturierte flexible Schicht dann vom Siliziumchip abgezogen. Zu den auf der Oberfläche der flexiblen Schicht entstandenen Siliziumnoppen wird schließlich über einzelne Anschlussleitungen ein Kontakt hergestellt. Nach einem ähnlichen Prinzip können rechteckige Gruben mit winzigen Abmessungen aus Polyurethan hergestellt werden, welche als Mikroküvetten für die Kultivierung von Nervenzellen fungieren können. Um einzelne Neuronen gezielt an Mikrosysteme anschließen zu können, werden auf der Oberfläche des Substrats unterstützende Strukturen, wie zum Beispiel grabenähnliche Mikrostrukturen vorgesehen. Ferner werden auf der Oberfläche Haftvermittler aufgetragen, welche das Aufwachsen von Zellen auf der Oberfläche des Substrats erleichtern. In solchen Strukturen wachsen ausgesäte Zellen zu netzartigen Gebilden heran und es bilden sich biohybride Systeme in Form zellenbewachsener Mikrochips aus. Als Haftvermittler an der Grenzfläche kommen Materialien in Betracht, die das Zellwachstum fördern und die Adhäsion der Zellen unterstützen.

Trotz der zahlreichen Aktivitäten auf dem Gebiet der Bioelektronik befindet sich dieses Gebiet noch in einem experimentellen Stadium, so dass insbesondere im Bereich der Schnittstelle zwischen elektronischem Bauteil und Zellen erhebliche Fortschritte erforderlich sind, um dieses Gebiet für eine medizinische Anwendung in der Praxis zugänglich zu machen.

Aufgabe der Erfindung ist es, ein Verfahren zur Herstellung biokompatibler Strukturen zur Verfügung zu stellen, welches einfach durchzuführen ist und die Herstellung einer Kontaktmatrix mit vielen Kontaktpunkten ermöglicht.

Die Aufgabe wird gelöst mit einem Verfahren zur Herstellung biokompatibler Strukturen, wobei ein chemisch verstärkter Fotoresist, der ein erstes Polymer enthält, welches Ankergruppen für die Anknüpfung einer biokompatiblen Verbindung umfasst, und ein zweites Polymer, welches elektrisch leitfähig ist, auf einem Substrat aufgetragen und strukturiert wird, so dass ein strukturierter Resist erhalten wird, und der strukturierte Resist mit einer biokompatiblen Verbindung behandelt wird, so dass die biokompatible Verbindung an die Ankergruppen des Polymers koordiniert wird.

Das erfindungsgemäße Verfahren nutzt eine Technik, wie sie bei der lithographischen Strukturierung von Halbleiterchips verwendet wird. Diese Technik ist sehr weit entwickelt, und es lassen sich mit ihrer Hilfe Strukturen bis hinab in den Bereich von weniger als 100 nm erzeugen. Für bioelektronische Anwendungen werden wie bereits oben erwähnt, Strukturen mit Abmessungen im Bereich von ca. 25 µm benötigt. Strukturen in diesen Abmessungen lassen sich daher mit den bekannten Fotoresists und Abbildungstechniken ohne weiteres darstellen. Das im Fotoresist verwendete erste Polymer muss lediglich Ankergruppen aufweisen, welche die nachträgliche Anbindung biokompatibler Substanzen ermöglicht. Eine nachträgliche Modifikation von Fotoresists ist bereits aus der Strukturierung von Halbleitern bekannt. Bei diesem Verfahren werden die auf einem Substrat erzeugten Resiststrukturen nachträglich durch Anknüpfung von Aufweitungsreagentien aufgeweitet, um auf diese Weise Strukturen herstellen zu können, deren Abmessungen unterhalb der Auflösungsgrenze der zur Belichtung verwendeten optischen Apparaturen liegen. Derartige Resists und Aufweitungsverfahren werden beispielsweise in der EP 395 917 B1 und der US 5,234,793 beschrieben (CARL: Chemical Amplification of Resist Lines).

Als elektrisch leitfähiges Polymer können die bekannten elektrisch leitfähigen Polymere eingesetzt werden. Diese müssen ggf. dotiert bzw. oxidiert oder reduziert werden, um sie in einen leitfähigen Zustand zu überführen. Dies kann auch in einem getrennten Schritt nach dem Auftrag des elektrisch leitfähigen Polymers auf das Substrat bzw. nach der Strukturierung des Resists erfolgen.

Für die Herstellung der biokompatiblen Strukturen können an sich alle chemisch verstärkten Fotoresists wie auch alle bekannten Strukturierungsverfahren eingesetzt werden. Erforderlich ist lediglich, dass am strukturierten Resist noch Gruppen vorhanden sind, welche die Anknüpfung einer biokompatiblen Verbindung ermöglichen. Es können sowohl positive wie auch negative chemisch verstärkte Fotoresists verwendet werden. Bei den positiven chemisch verstärkten Resists werden im Entwicklungsschritt die belichteten Abschnitte des Fotoresists mit einer Entwicklungslösung entfernt, während die unbelichteten Bereiche als Stege auf dem Substrat verbleiben. Dies wird dadurch erreicht, dass durch die Belichtung ein Katalysator freigesetzt wird, welcher das Polymer des Fotoresists in seiner chemischen Natur so verändert, dass eine deutliche Differenzierung zwischen belichteten und unbelichteten Bereichen erreicht wird. Dies kann beispielsweise dadurch erreicht werden, dass Gruppen am Polymer abgespalten werden, wodurch sich die Polarität des Polymers deutlich erhöht, so dass es in wässrigen Entwicklern löslich wird. Es können auch negativ strukturierbare Resists verwendet werden, bei denen die belichteten Bereiche auf dem Substrat als Stege verbleiben, während die unbelichteten Bereiche mit einem wässrigen Entwickler entfernt werden. Die chemische Differenzierung zwischen unbelichteten und belichteten Abschnitten wird dabei meist in der Weise durchgeführt, dass durch die Belichtung ein Katalysator freigesetzt wird, welcher beispielsweise eine Vernetzung des Polymers des Fotoresists bewirkt, wodurch dieses in wässrigen Entwicklern unlöslich wird. Im Entwicklungsschritt werden dann die unbelichteten Bereiche, die meist Verbindungen mit hoher Polarität aufweisen, mit einem wässrigen Entwickler entfernt. Es konnen auch modifizierte Verfahren angewendet werden, die auf den oben erwähnten positiven und negativen chemisch verstärkten Fotoresistsystemen beruhen. Ein derartiges Verfahren ist beispielsweise in der US 4,491,628 beschrieben. Dabei wird die auf einem Substrat aufgetragene Schicht eines positiven Fotoresists zunächst belichtet, wobei aus einem Fotosäurebildner eine Säure freigesetzt wird. Im anschlieβenden Verstärkungsschritt werden durch Tempern in den belichteten Bereichen säurelabile Gruppen vom Polymer abgespalten, so dass dieses nun in einer polaren Form vorliegt. Im Unterschied zum oben beschriebenen positiven Entwicklungsverfahren wird nun nicht mit einem polaren wässrigen Entwickler entwickelt, sondern es wird für die Entwicklung ein unpolares Lösungsmittel verwendet. Dadurch werden nur die unbelichteten Bereiche vom Substrat abgelöst, in denen das Polymer seine ursprüngliche unpolare Form beibehalten hat. Da die polaren Anteile des Resists, in denen durch die Belichtung polare Gruppen erzeugt wurden, beispielsweise Carbonsäuregruppen, in unpolaren Lösungsmitteln unlöslich sind, verbleiben diese als Stege auf dem Substrat.

Für die Herstellung eines strukturierten Resists kann auch ein Verfahren angewandt werden, wie es beispielsweise in der PCT/DE00/04237 beschrieben ist. Dabei enthält der Fotoresist eine Fotobase wie auch eine Thermosäure. Bei der Belichtung des Fotoresists wird in den belichteten Bereichen eine Base freigesetzt. Wird der Fotoresist anschließend erwärmt, so wird aus dem Thermosäurebildner eine Säure freigesetzt. In den belichteten Bereichen wird die Säure durch die zuvor freigesetzte Base neutralisiert und steht daher nicht mehr als Katalysator zur Verfügung. In den unbelichteten Bereichen katalysiert die Säure die Abspaltung säurelabiler Gruppen aus dem Polymer. Das Polymer wird daher in den unbelichteten Bereichen von seiner unpolaren Form in eine polare Form überführt. Im anschließenden Entwicklerschritt können daher die unbelichteten Bereiche mit einem wässrig-alkalischen Entwickler selektiv vom Substrat abgelöst werden, während die belichteten Bereiche als Stege auf dem Substrat verbleiben.

Bei allen diesen Verfahren ist es wesentlich, dass nach der Strukturierung des Fotoresists noch Gruppen für die Anbindung der biokompatiblen Verbindung zur Verfügung stehen.

Bevorzugt wird für die Strukturierung des Fotoresists jedoch ein Verfahren eingesetzt, wie es in der EP 0 395 917 Bl beschrieben ist. Dabei wird ein positiver Fotoresist verwendet, an welchem nach dem Belichten, Verstärken und Entwikkeln in einem weiteren Schritt die biokompatible Verbindung angeknüpft wird.

Das Verfahren umfasst in dieser Ausführungsform die folgenden Schritte:
Aufbringen eines Fotoresistfilms auf ein Substrat, wobei der Fotoresistfilm die folgenden Komponenten enthält:
ein erstes Polymer, welches säurelabile Gruppen umfasst, die nach ihrer Abspaltung eine polare Gruppe freisetzen, wodurch die Löslichkeit des ersten Polymers in wässrig-alkalischen Entwicklern erhöht wird, und das weiter Ankergruppen für die Anknüpfung einer biokompatiblen Verbindung aufweist, wobei die Ankergruppen auch in geschützter Form vorliegen können;
einen Fotosäurebildner;
ein zweites Polymers, welches elektrisch leitfähig ist, wobei erstes und zweites Polymer als Mischung oder als aufeinander angeordnete Schichten auf dem Substrat angeordnet sein können;
abschnittsweises Belichten des Fotoresistfilms;
Tempern des belichteten Fotoresistfilms, wobei in den belichteten Abschnitten die säurelabilen Gruppen vom Polymer abgespalten werden;
Entwickeln des belichteten und getemperten Fotoresistfilms mit einer wässrig-alkalischen Entwicklerlösung, wobei die belichteten Abschnitte des Fotoresistfilms vom Substrat abgelöst werden und ein strukturierter Resist erhalten wird;
gegebenenfalls Ätzen der Schicht des elektrischen leitfähigen zweiten Polymers;
gegebenenfalls Freisetzen der Ankergruppen des ersten Polymers;
Aufbringen einer Lösung der biokompatiblen Verbindung, wobei die biokompatible Verbindung an die Ankergruppen des ersten Polymers koordiniert wird;
Entfernen überschüssiger Lösung der biokompatiblen Verbindung.

Diese Ausführungsform des erfindungsgemäßen Verfahrens kann auf zwei Wegen ausgeführt werden, wobei das erste und das zweite Polymer entweder in einer gemeinsamen Schicht oder in zwei voneinander getrennten Schichten enthalten ist. Beim zuerst genannten Weg wird eine Lösung auf das Substrat aufgebracht, welche das erste Polymer, das zweite Polymer und den Fotosäurebildner in einem geeigneten Lösungsmittel enthält. Durch Verdampfen des Lösungsmittels wird ein fotostrukturierbarer Film erhalten, der mit gängigen lithographischen Verfahren strukturiert wird. Anschließend wird wie oben beschrieben die biokompatible Verbindung aufgebracht, welche an die Ankergruppen des ersten Polymers koordiniert wird.

Gemäß dem zweiten Weg werden erstes und zweites Polymer in getrennten Schichten auf das Substrat aufgebracht. Dabei wird zunächst eine erste Schicht des zweiten, elektrisch leitfähigen, Polymers auf dem Substrat erzeugt. Auf diesem wird dann eine zweite Schicht erzeugt, die das erste Polymer sowie den Fotosäurebildner enthalt. Anschließend wird die zweite Schicht des ersten Polymers belichtet und entwickelt und dadurch strukturiert. Diese Struktur wird anschließend in die erste Schicht, welche das elektrisch leitfähige zweite Polymer enthält, übertragen, wozu beispielsweise ein geeignetes Plasma verwendet wird. Anschließend wird wiederum die biokompatible Verbindung aufgebracht, welche an die Ankergruppen des ersten Polymers koordiniert wird.

Nach einer weiteren bevorzugten Ausführungsform des Verfahrens wird auf dem Substrat eine Schicht erzeugt, die das erste Polymer und den Fotosäurebildner umfasst. Die Schicht wird anschließend abschnittsweise belichtet und entwickelt und dadurch eine strukturierte Schicht erhalten. Auf die strukturierte Schicht wird anschließend eine Schicht des zweiten Polymers aufgebracht.

Bei dieser Ausführungsform wird das Verfahren bevorzugt in der Weise geführt, dass das zweite Polymer in das erste Polymer eindringen kann. Dazu kann das zweite Polymer als Lösung in einem geeigneten Lösungsmittel auf die strukturierte Schicht aufgebracht werden. Das Lösungsmittel muss dazu so beschaffen sein, dass es einerseits die Struktur der strukturierten Schicht nicht auflöst, andererseits das erste Polymer jedoch soweit anlöst oder quillt, dass das zweite Polymer ausreichend tief eindringen kann.

Vorzugsweise wird das zweite Polymer an das erste Polymer koordiniert, beispielsweise über die im ersten Polymer vorgesehenen Ankergruppen.

Als erstes Polymer können für den Fotoresist solche Polymere eingesetzt werden, welche nach der Entwicklung noch Gruppen aufweisen, an welche die biokompatible Verbindung koordinieren kann. Die ersten Polymere müssen ausreichende Filmbildungseigenschaften aufweisen, um einen gleichmäßigen Film des Fotoresists auf dem Substrat erzeugen zu können. Es können alle Polymere eingesetzt werden, die in der Polymerkette oder seitenständig säurelabile Gruppen mit geringer Alkalilöslichkeit besitzen, welche durch katalytische Einwirkung von Säure und gegebenenfalls einer gleichzeitigen Temperaturbehandlung (Kontrastierung) polare Gruppen, zum Beispiel saure Gruppen, am Polymer erzeugen. Als säurelabile Gruppen kommen zum Beispiel in Betracht: tert.-Alkylester-, tert.-Butoxycarbonyloxy-, Tetrahydrofuranyloxy-, Tetrahydropyranyloxy-, tert.-Butylether-, Lacton- oder Acetalgruppen. Tert.-Butylestergruppen sind besonders bevorzugt.

Das filmbildende Polymer kann daher durch Polymerisation oder Copolymerisation entsprechender Monomere erhalten werden. Als Monomere sind beispielsweise Acrylate, Methacrylate, Maleinsäuremono- und -diester, Itaconsäuremono- und - diester, Norbornencarbonsäureester oder auch Norbonendicarbonsäuremono- und -diester geeignet. Entsprechende Wiederholungseinheiten des Polymers sind im Weiteren dargestellt. Y steht dabei für einen durch Säure abspaltbaren Rest, wie er beispielsweise in einer der oben genannten Gruppen enthalten ist und nach dessen Abspaltung die polare Gruppe, beispielsweise eine Carboxyl- oder eine Hydroxylgruppe freigesetzt wird, und R¹ für einen nicht säurelabilen Rest, beispielsweise für eine Alkylgruppe mit 1 bis 10 Kohlenstoffatomen. Ferner bezeichnet n eine ganze Zahl zwischen 1 und 10.

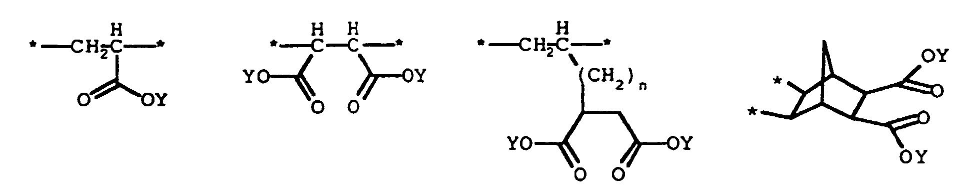

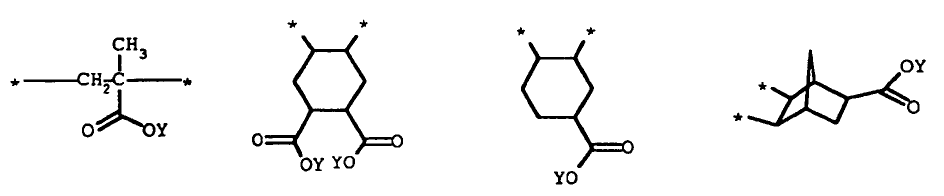

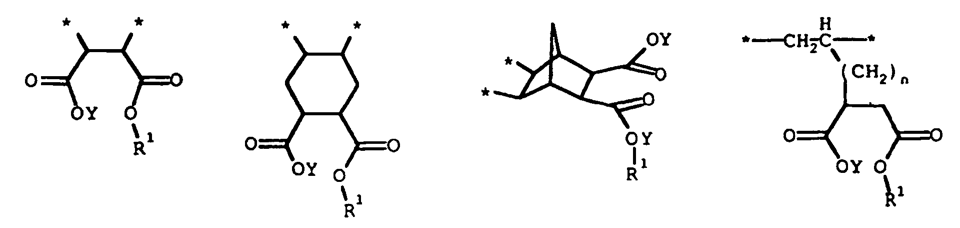

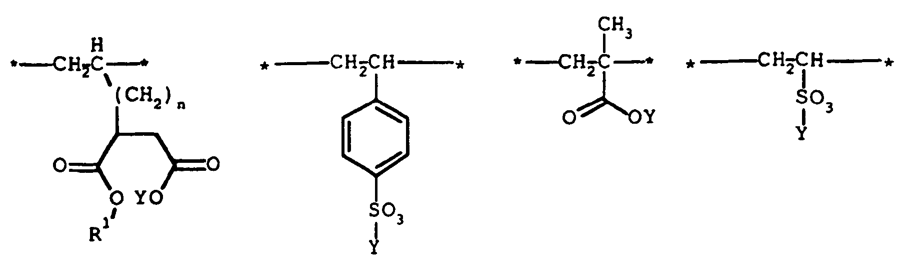

Die Abspaltung des säurelabilen Restes aus der säurelabilen Gruppe unter Freisetzung der polaren Gruppe ist im Folgenden beispielhaft an zwei bevorzugten Wiederholungseinheiten dargestellt. Im ersten Beispiel umfasst die Wiederholungseinheit eine tert.-Butylestergruppe, aus der unter Einwirkung von Säure eine Carboxylgruppe freigesetzt wird.

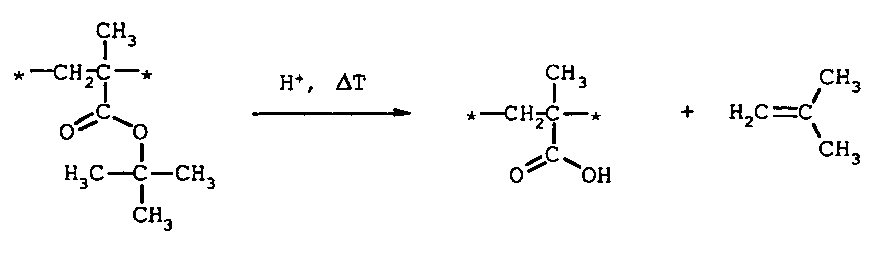

Im zweiten Beispiel umfasst die säurelabile Gruppe einen tert.-Butoxycarbonyloxyrest, welcher an eine phenolische Hydroxylgruppe gebunden ist. Unter Einwirkung von Säure wird als polare Gruppe daher eine saure Hydroxylgruppe freigesetzt.

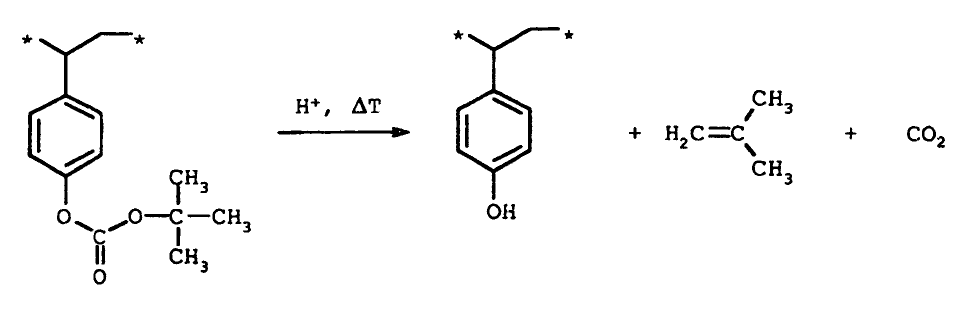

Diese Monomeren können mit weiteren Monomeren copolymerisiert werden. Ein geeignetes Monomer ist beispielsweise Styrol. Neben den genannten Monomeren können auch andere für die Herstellung von in Fotoresists enthaltenen Polymeren übliche Monomere verwendet werden. Beispielsweise können cycloaliphatische Gruppen durch Copolymerisation von Norbornen und Norbornenderivaten eingeführt werden. Siliziumhaltige Gruppen lassen sich durch Copolymerisation von Trialkylallylsilanen einführen. Die genaue Zusammensetzung des Polymeren hängt von den Eigenschaften ab, die für die weitere Prozessierung gefordert werden. Wird der Fotoresist zum Beispiel auch zum Ätzen des Substrats verwendet, so muss dieser eine ausreichende Ätzresistenz aufweisen. Dies wird erreicht, indem in das Polymer siliziumhaltige Gruppen, aromatische Gruppen oder alicyclische Gruppen eingeführt werden.

Als Fotosäurebildner können die für Fotoresists üblichen Fotorsäurebildner eingesetzt werden. Bevorzugt werden Oniumverbindungen verwendet, wie sie beispielsweise in der EP 0 955 562 A1 beschrieben sind.

Als Lösungsmittel des Resists kann zum Beispiel Methoxypropylacetat, Cyclopentanon, Cyclohexanon, γ-Butyrolacton, Ethyllactat, Diethylenglykoldimethylether oder ein Gemisch aus wenigstens zwei dieser Verbindungen verwendet werden. Allgemein können aber alle gängigen Lösungsmittel oder deren Gemische verwendet werden, die in der Lage sind, die Resistkomponenten in einer klaren, homogenen und lagerstabilen Lösung aufzunehmen, die bei der Beschichtung des Substrates eine gute Schichtqualität gewährleisten.

Der Fotoresist wird mit den gängigen Verfahren auf dem Substrat aufgetragen, zum Beispiel durch Aufschleudern, Aufsprühen, Tauch- oder Pinselverfahren. Anschließend wird das Lösungsmittel mit gängigen Verfahren entfernt. Dazu wird im Allgemeinen das Substrat mit dem Resistfilm erwärmt.

Das zweite Polymer, welches elektrisch leitend ist, kann im Gemisch mit dem ersten Polymer aufgebracht werden oder als getrennte Schicht. Viele elektrisch leitfähige Polymere müssen so abgeschieden werden, dass eine hohe kristalline Ordnung im abgeschiedenen Feststoff erreicht wird. In diesem Fall erfolgt die Abscheidung des elektrisch leitfähigen zweiten Polymers in einer getrennten Schicht, wobei die Schicht des ersten Polymers auf der Schicht des zweiten Polymers angeordnet ist. Das zweite Polymer kann bereits bei seiner Abscheidung elektrisch leitfähig sein oder erst nachträglich durch Oxidation oder Reduktion in seinen leitfähigen Zustand überführt werden.

Anschließend erfolgt ein Belichten des Resistfilms, wozu ebenfalls die gängigen Verfahren angewandt werden können. Die Belichtung kann beispielsweise mittels einer Fotomaske erfolgen oder auch durch direkte Belichtung mit fokussierten Elektronen oder Ionen. Bevorzugt weist die Belichtungsstrahlung eine Wellenlänge im Bereich von 10 bis 400 nm auf. Da für die biokompatiblen Strukturen keine besonders hohe Auflösung gefordert ist, wird üblicherweise Licht einer Wellenlänge von 365 nm, 248 nm oder 193 nm verwendet, wie es auch bei der Herstellung von Mikrochips verwendet wird. In den belichteten Bereichen wird aus dem Fotosäurebildner eine Säure freigesetzt, wobei ein latentes Bild der gewünschten Struktur gebildet wird. Nach dem Belichten des Resistfilms erfolgt ein Kontrastierungsschritt, in dem das latente Bild verstärkt und in das Polymer des Fotoresists eingeprägt wird, so dass der Fotoresist nur ein chemisches Profil aufweist. Dazu wird das Substrat mit dem belichteten Resistfilm erhitzt, im allgemeinen auf Temperaturen von 80 bis 200°C. Beim Tempern werden unter dem katalytischen Einfluss der Säure die säurelabilen Gruppen am Polymer abgespalten und polare Gruppen freigesetzt. Das Polymer weist nun eine hohe Polarität und damit eine Löslichkeit in polaren Lösungsmitteln auf. Die belichteten Bereiche können daher mit einer wässrig-alkalischen Entwicklerlösung abgelöst werden. Als Entwicklerlösung kann beispielsweise eine 2,38 %-ige Lösung aus Tetramethylammoniumhydroxid in Wasser verwendet werden. Nach der Entwicklung wird ein strukturierter Resist erhalten, welcher die Strukturen aufweist, an die später die Zellen angebunden werden sollen. Je nachdem, welches zweite Polymer verwendet wird, kann erforderlich sein, dass die mit dem ersten Polymer erzeugte Struktur in die Schicht des zweiten Polymers übertragen wird. Dazu kann beispielsweise ein Ätzschritt durchgeführt werden, in dem das zweite Polymer in den Bereichen, in denen es nicht vom ersten Polymer bedeckt ist, entfernt wird.

Um die biokompatible Verbindung in den strukturierten Fotoresist einführen zu können, muss das Polymer entsprechende Ankergruppen für die Anknüpfung einer biokompatiblen Verbindung aufweisen. Dazu kann in der Weise vorgegangen werden, dass der strukturierte Fotoresist, welcher noch säurelabile Gruppen umfasst, flutbelichtet wird. Dabei wird in den zuvor unbelichteten Bereichen nun ebenfalls eine Säure freigesetzt. Unter Erwärmen werden nun ebenfalls die säurelabilen Gruppen abgespalten und polare Gruppen, zum Beispiel Carboxylgruppen oder saure alkoholische Gruppen, wie zum Beispiel saure phenolische Gruppen, freigesetzt. Diese können dann als Ankergruppen für die Koordination der biokompatiblen Verbindung verwendet werden. Es ist auch möglich, im Fotoresist zusätzlich einen Thermosäurebildner vorzusehen. Der strukturierte Fotoresist kann dann erhitzt werden, wobei die Säure freigesetzt wird und ebenfalls die Abspaltung der säurelabilen Gruppen bewirkt wird.

Nach der Freisetzung der Ankergruppen durch die Abspaltung der säurelabilen Gruppen wird eine Lösung der biokompatiblen Verbindung auf den strukturierten Resist aufgetragen. Das Lösungsmittel ist dabei so ausgewählt, dass der strukturierte Resist nicht vom Substrat abgelöst wird und gleichzeitig die biokompatible Verbindung vom Lösungsmittel in Form einer Lösung oder einer Emulsion aufgenommen wird. Geeignet sind z.B. gepufferte wässrige Lösungen. Die biokompatible Verbindung, welche eine geeignete Koordinationsgruppe aufweist, koordiniert nun an die Ankergruppen des Polymeren. Dies muss nicht notwendigerweise unter Ausbildung einer kovalenten Bindung erfolgen. Die Koordination der biokompatiblen Verbindung an die Ankergruppe des Polymers kann auch unter Salzbildung erfolgen, indem beispielsweise die Ankergruppen am Polymer durch Carboxylgruppen und die koordinative Gruppe an der biokompatiblen Verbindung durch eine Aminogruppe gebildet wird. Es ist auch einen Koordination durch Dipol-Dipol-Wechselwirkungen möglich, sofern eine ausreichend starke Fixierung der biokompatiblen Verbindung im Fotoresist erfolgt. Eine solche Koordination der biokompatiblen Verbindung an die Ankergruppe des Polymers durch nicht-kovalente Bindungen hat den Vorteil, dass die Bindung reversibel ist. Werden beispielsweise Wachstumsfaktoren als biokompatible Verbindung auf den strukturierten Resist gegeben, so können diese von den Zellen aufgenommen werden und so das Zellwachstum beeinflusst werden.

In einigen Fällen kann es bevorzugt sein, dass die biokompatible Verbindung durch eine kovalente Bindung an das Polymer des Fotoresists gebunden wird. Die Ausbildung einer kovalenten Bindung kann in einem nachgeschalteten Schritt erfolgen, indem der Fotoresist erwärmt wird und beispielsweise aus einem Ammoniumcarboxylat unter Wasserabspaltung eine Amidbindung ausgebildet wird.

Die Koordination der biokompatiblen Verbindung unter Ausbildung einer kovalenten Bindung kann auch durch eine Reaktion mit geeigneten Gruppen des Polymers erfolgen. Dazu sind die Ankergruppen für die Anbindung der biokompatiblen Verbindung im Polymer als Reaktivankergruppe ausgebildet. Unter einer Reaktivankergruppe wird eine Ankergruppe im Polymer verstanden, an welche die biokompatible Verbindung unter Ausbildung einer kovalenten Bindung koordiniert wird. Die Reaktivankergruppen weisen eine ausreichende Reaktivität auf, um innerhalb hinreichend kurzer Reaktionszeiten eine chemische Bindung zur biokompatiblen Verbindung ausbilden zu können. Geeignete Gruppen sind beispielsweise Carbonsäureanhydride, Epoxide, Isocyanate, Glycidylether, Amine, Alkylhalogenide, Thiole sowie Acylhalogenide.

Insbesondere bevorzugt umfasst das Polymer als Reaktivankergruppen Carbonsäureanhydridgruppen. Diese können in das Polymer eingeführt werden, indem beispielsweise Maleinsäureanhydrid, Itaconsäureanhydrid, Methacrylsäureanhydrid, Cyclohexendicarbonsäureanhydrid oder Norbornendicarbonsäureanhydrid bei der Herstellung des Polymers copolymerisiert werden. Beispielhafte Wiederholungseinheiten des Polymers, welche eine Dicarbonsäureanhydridfunktion aufweisen, sind im Folgenden dargestellt: 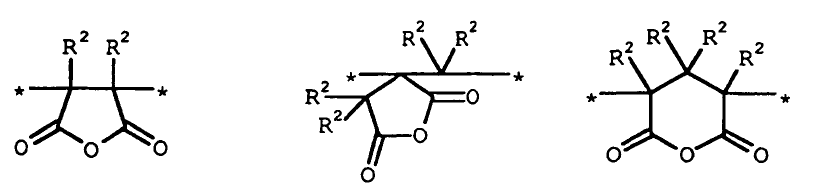 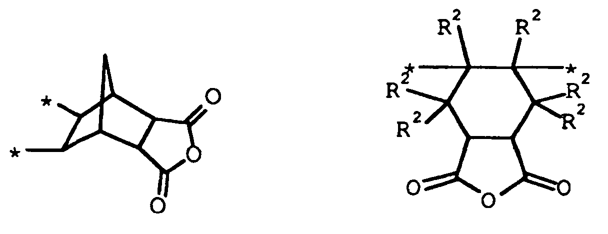 R² steht dabei bevorzugt für Wasserstoff oder für einen beliebigen anderen Rest, insbesondere für einen Alkylrest mit 1 bis 10 Kohlenstoffatomen. Die Reste R² können dabei unabhängig voneinander die genannte Bedeutung aufweisen.

Wie bereits erwähnt, muss die biokompatible Verbindung für die Koordination an eine Ankergruppe des Polymeren eine geeignete Koordinationsgruppe aufweisen. Besonders bevorzugt umfasst die biokompatible Verbindung eine Aminogruppe und/oder eine Hydroxylgruppe, über welche die biokompatible Verbindung an die Ankergruppe des Polymers koordiniert wird.

Die Koordination kann dabei über eine einzelne Gruppe oder auch über mehrere Gruppen erfolgen. Die Koordination erfolgt beispielsweise durch die Reaktion der Aminogruppe mit einem Carbonsäureanhydrid unter Ausbildung einer Amidbindung oder einer Imidogruppe. Wird eine Hydroxylgruppe der biokompatiblen Verbindung für die Koordination verwendet, wird entsprechend eine Estergruppe ausgebildet. Derartige Bindungen können von Zellen enzymatisch gespalten werden, so dass die biokompatible Verbindung während des Aufwachsens der Zelle von dieser wieder vom Resist abgespalten werden kann.

Als biokompatible Verbindung können an sich alle Verbindungen verwendet werden, welche ein Aufwachsen von Zellen auf die aus dem Fotoresist erzeugten Strukturen erleichtern. Diese Verbindungen können zum Teil hochmolekulare biologische Verbindungen sein. In einer bevorzugten Ausführungsform umfasst die biokompatible Verbindung einen Spacer, welcher die Aminogruppe oder die Hydroxylgruppe für die Koordination der biokompatiblen Verbindung an die Ankergruppe des Polymers trägt. Dies erleichtert eine Anbindung von hochmolekularen Verbindungen an das Polymer, da die sterische Hinderung verringert wird. Außerdem verbessert sich die Wirkung der biokompatiblen Verbindung, wenn diese über einen Spacer von der Oberfläche des strukturierten Resists beabstandet ist. Techniken für die Immobilisierung hochmolekularer Verbindungen auf Oberflächen sind beispielsweise aus der Immobilisierung von Antigenen auf hochmolekularen Proteinen bekannt. Entsprechende Techniken können auch für die Koordination der biokompatiblen Verbindung an die Ankergruppen des Polymeren verwendet werden.

Als biokompatible Verbindung wird vorzugsweise eine Aminosäure oder ein Peptid verwendet. Diese Verbindung enthalten bereits Gruppen, z.B. Aminogruppen, welche für die Koordination der biokompatiblen Verbindung an das Polymer des Resists verwendet werden können. Als Peptide können beispielsweise Wachstumsfaktoren verwendet werden. Die Aminosäure oder das Peptid kann auch wiederum als Koordinationsstelle verwendet werden, an die entsprechende wachstumsfördernde Faktoren reversibel gebunden werden. Beim Aufwachsen der Zellen werden diese Faktoren dann von der Oberfläche des strukturierten Resists abgelöst und von den Zellen aufgenommen.

Gemäß einer weiteren vorteilhaften Ausführungsform ist die biokompatible Verbindung ein oligomeres oder polymeres Urethan. Urethane können von Zellen enzymatisch gespalten werden. Derartige Verbindungen fördern daher das Wachstum von Zellen.

Um eine bessere Übertragung elektrischer Signale zwischen Zellen und elektronischem Bauteil zu erreichen, umfasst die Resiststruktur als zweites Polymer ein elektrisch leitfähiges Polymer. Dabei können an sich alle elektrisch leitfähigen organischen Materialien verwendet werden. Beispielsweise kann das elektrisch leitfähige Polymer ausgewählt werden aus der Gruppe, die gebildet ist aus elektrisch leitfähigem Polyanilin, elektrisch leitfähigem Polythiophen, elektrisch leitfähigem Polypyrrol und elektrisch leitfähigem Polyvinylphenylen.

Als Substrat können an sich alle Materialien verwendet werden, welche mit dem Aufwachsen von Zellen verträglich sind. So können beispielsweise polymere Materialien verwendet werden oder auch Halbleiter, wie zum Beispiel Silizium. Da der strukturierte Resist für eine Informationsübertragung zwischen Mikroelektronik und Zellen eingesetzt werden soll, beispielsweise durch eine Übertragung elektrischer Signale, ist das Substrat vorzugsweise ein Mikrochip. Dieser kann entsprechende mikroelektronische Schaltungen umfassen.

Die Erfindung umfasst auch einen biokompatiblen Mikrochip, umfassend:
ein Substrat, welches mikroelektronische Schaltungen umfasst, sowie eine auf dem Substrat angeordnete strukturierte Schicht, welche ein erstes Polymer enthält, das Ankergruppen aufweist, an die eine biokompatible Verbindung koordiniert ist, sowie ein zweites Polymer, das elektrisch leitfähig ist.

Gemäß einer bevorzugten Ausführungsform weist der biokompatible Mikrochip eine Struktur auf, bei der das erste Polymer und das zweite Polymer in getrennten Schichten angeordnet ist. Vorzugsweise ist das erste Polymer in einer Schicht enthalten, die auf der Schicht des zweiten Polymers angeordnet ist.

Die Erfindung wird unter Bezugnahme auf eine beigefügte Zeichnung näher erläutert. Dabei zeigt:
Fig. 1: Eine Abfolge der Verfahrensschritte gemäß einer ersten bevorzugten Ausführungsform des erfindungsgemäßen Verfahrens;
Fig. 2: Eine Abfolge der Verfahrensschritte gemäß einer zweiten bevorzugten Ausführungsform des erfindungsgemäßen Verfahrens;
Fig. 3: Eine Abfolge der Verfahrensschritte gemäß einer dritten bevorzugten Ausführungsform des erfindungsgemäßen Verfahrens.

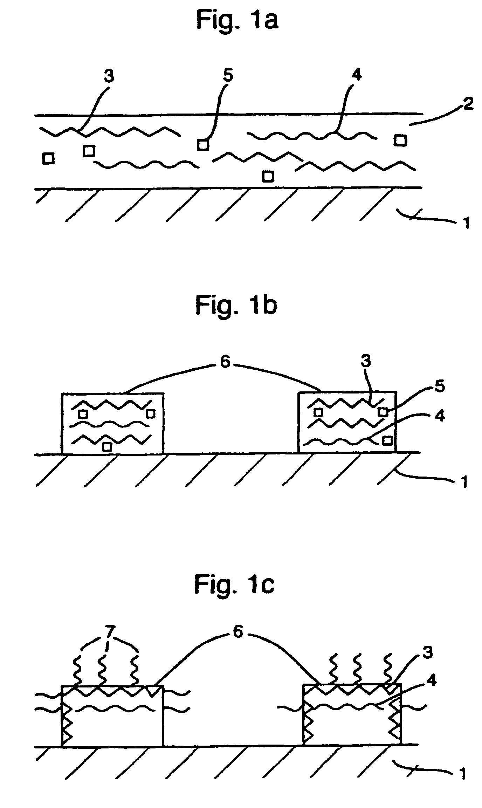

In Fig. 1 sind die Verfahrensschritte dargestellt, die durchlaufen werden, wenn erstes und zweites Polymer in einer gemeinsamen Schicht enthalten sind. Dazu wird wie in Fig. 1a gezeigt, auf einem Substrat 1, beispielsweise ein Mikrochip, in welchem bereits elektronische Schaltkreise integriert sind, ein Fotoresistfilm 2 aufgebracht. Im Fotoresistfilm 2 sind das erste Polymer 3, das zweite Polymer 4 sowie der Fotosäurebildner 5 enthalten. Der Fotoresistfilm 2 wird anschließend abschnittsweise belichtet und entwickelt, sodass der in Fig. 1b dargestellte strukturierte Resist erhalten wird. Dieser umfasst auf dem Substrat 1 angeordnete erhabene Abschnitte 6, welche aus dem Material des Fotoresistfilms 2 gebildet sind und daher das erste Polymer 3, das zweite Polymer 4 und den Fotosäurebildner 5 enthalten. Anschließend wird eine Lösung der biokompatiblen Verbindung aufgebracht, die an das erste Polymer 3 koordiniert wird. Dies kann unmittelbar nach der Strukturierung des Fotoresistfilms erfolgen, sofern das erste Polymer 3 entsprechende Ankergruppen aufweist. Andernfalls wird zunächst flutbelichtet, sodass aus dem noch vorhandenen Fotosäurebildner 5 Säure freigesetzt wird. Anschließend wird zur Spaltung der säurelabilen Gruppen getempert, wobei als Ankergruppen wirkende polare Gruppen freigesetzt werden. Die Koordination der biokompatiblen Verbindung 7 an die Ankergruppen des ersten Polymers 3 ist schematisch in Fig. lc dargestellt. An den Außenflächen der erhabenen Abschnitte 6 hat sich die biokompatible Verbindung 7 angelagert, welche durch Koordination an das erste Polymer 3 fixiert wird. Auf dieseStruktur können anschlieβend Zellen ausgesät werden.

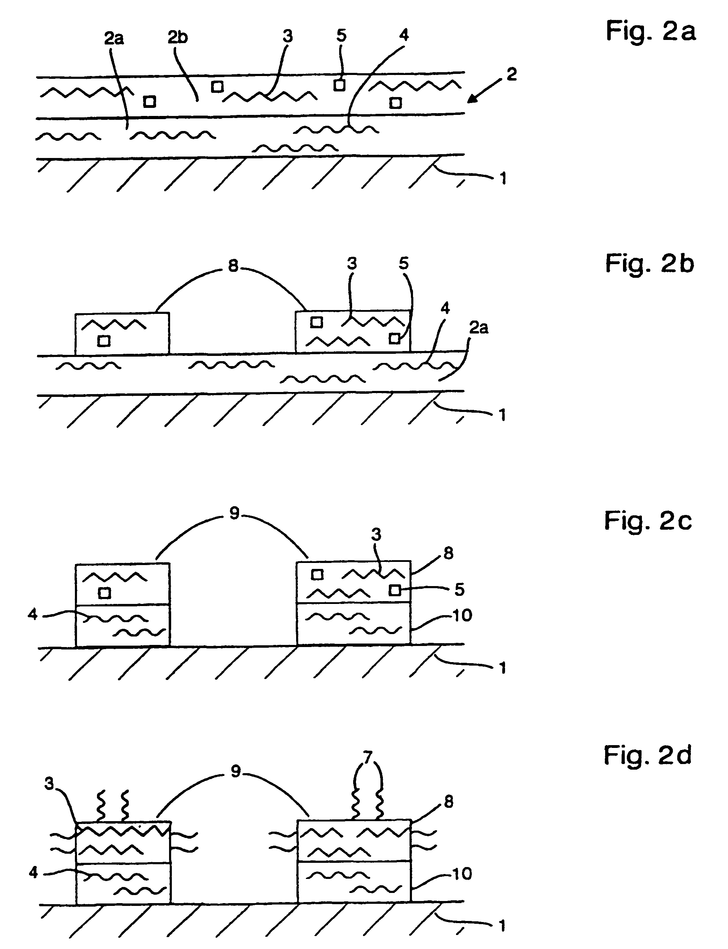

Bei der in Fig. 2 gezeigten Ausführungsform des erfindungsgemäßen Verfahrens ist das erste und das zweite Polymer in getrennten Schichten angeordnet. Dazu wird zunächst wie in Fig. 2a gezeigt, ein zweilagiger Fotoresistfilm 2 erzeugt, der eine auf dem Substrat 1 angeordnete erste Schicht 2a des zweiten, elektrisch leitfähigen Polymers 4 und eine auf dieser ersten Schicht 2a angeordnete zweite Schicht 2b umfasst, welche das erste Polymer 3 und den Fotosäurebildner 5 enthält. Anschließend wird der Fotoresistfilm 2 abschnittsweise belichtet und entwickelt, sodass die zweite Schicht 2b strukturiert wird. Dieser Zustand ist in Fig. 2b dargestellt. Auf der Schicht 2a sind erhabene Abschnitte 8 entstanden, welche das erste Polymer 3 und den Fotosäurebildner 5 enthalten. Die durch die erhabenen Abschnitte 8 gebildete Struktur wird nun in die erste Schicht 2a des elektrisch leitfähigen zweiten Polymers 4 übertragen. Wie in Fig. 2c dargestellt sind nun auf dem Substrat 1 erhabene Abschnitte 9 angeordnet, welche in ihrem oberen Abschnitt von den Abschnitten 8 des ersten Polymers 3 und in ihrem unterem Abschnitt vom Abschnitt 10 des zweiten Polymers 4 gebildet werden. Es können nun ggf. die Ankergruppen des ersten Polymers 3 freigesetzt und dann eine Lösung der biokompatiblen Verbindung 7 aufgebracht werden, die an die Moleküle des ersten Polymers 3 koordiniert. Wie in Fig. 2d dargestellt, sind an den Außenflächen des Abschnitts 8 der erhabenen Strukturen 9 biokompatible Moleküle 7 koordiniert. Anschlieβend kann die Struktur wieder von Zellen besiedelt werden.

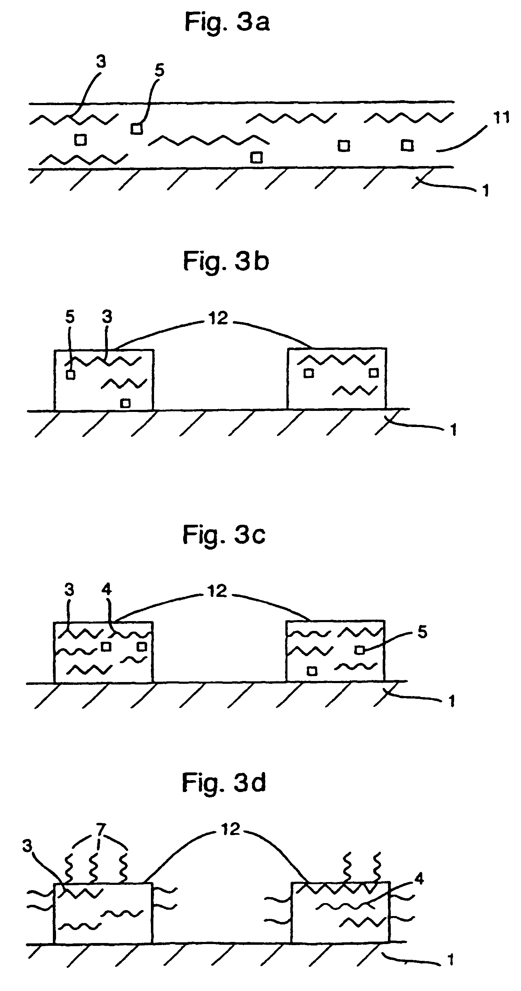

Bei der n Fig. 3 gezeigten Verfahrensabfolge wird zunächst eine Resiststruktur aus dem ersten Polymer 3 erzeugt und das elektrisch leitfähige zweite Polymer 4 nachträglich eingeführt. Dazu wird zunächst auf dem Substrat 1 eine Schicht 11 erzeugt, die das erste Polymer 3 und den Fotosäurebildner 5 enthält. Diese in Fig. 3a gezeigte Anordnung wird nun abschnittsweise belichtet und entwickelt, sodass die in Fig. 3b gezeigte Anordnung erhalten wird. Auf dem Substrat 1 sind erhabene Bereiche 12 angeordnet, die das erste Polymer 3 und den Fotosäurebildner 5 enthalten. Es wird nun eine Lösung des zweiten Polymers 4 in einem geeigneten Lösungsmittel aufgebracht. Das Lösungsmittel quillt oder löst die erhabenen Abschnitte 12 an, sodass das zweite Polymer 4 eindringen kann. Da die erhabenen Abschnitte 12 relativ große Abmessungen aufweisen und auch der Strukturerhalt dieser Abschnitte 12 beim Einführen des zweiten Polymers 4 nicht kritisch ist, kann dabei ein geringfügiges Ablösen an den erhabenen Abschnitten 12 in Kauf genommen werden. Anschließend wird wiederum, ggf. nach Freisetzen der Ankergruppen am ersten Polymer 3, die biokompatible Verbindung als Lösung aufgegeben sodass, wie in Fig. 3d gezeigt, die biokompatible Verbindung 7 an die Außenflächen der erhabenen Bereiche 12 koordiniert werden kann.

Die Erfindung stellt ein Verfahren zur Verfügung, das auf lithographischen Verfahren beruht, die bei der Herstellung von Mikrochips bereits industriell eingesetzt werden. Zur Verfahrensführung steht daher bereits ein großes Wissen zur Verfügung. Strukturen in den für bioelektronische Anwendungen erforderlichen Dimensionen lassen sich mit diesen Verfahren ohne weiteres herstellen. Der strukturierte Resist verbleibt auf dem Substrat und wird nach einer entsprechenden Konditionierung durch eine biokompatible Verbindung mit Zellen bewachsen. Durch das in der strukturierten Resistschicht enthaltene elektrisch leitfähige Polymer wird ein verbesserter Kontakt zu den Zellen hergestellt, was eine bessere Signalübertragung ermöglicht.

## Patentansprüche

1. Verfahren zur Herstellung biokompatibler Strukturen, wobei ein chemisch verstärkter Fotoresistfilm, der ein erstes Polymer enthält, welches Ankergruppen für die Anknüpfung einer biokompatiblen Verbindung umfasst, und ein zweites Polymer, welches elektrisch leitfähig ist, auf einem Substrat aufgebracht und strukturiert wird, so dass ein strukturierter Resist erhalten wird, und der strukturierte Resist mit einer biokompatiblen Verbindung behandelt wird, so dass die biokompatible Verbindung an die Ankergruppen des ersten Polymers koordiniert wird.

2. Verfahren nach Anspruch 1, umfassend die Schritte:
Aufbringen eines Fotoresistfilms auf ein Substrat, wobei der Fotoresistfilm die folgenden Komponenten enthält:
ein erstes Polymer, welches säurelabile Gruppen umfasst, die nach ihrer Abspaltung eine polare Gruppe freisetzen, wodurch die Löslichkeit des ersten Polymers in wässrig-alkalischen Entwicklern erhöht wird, und das weiter Ankergruppen für die Anknüpfung einer biokompatiblen Verbindung aufweist, wobei die Ankergruppen auch in geschützter Form vorliegen können;
einen Fotosäurebildner;
ein zweites Polymers, welches elektrisch leitfähig ist, wobei erstes und zweites Polymer als Mischung oder als aufeinander angeordnete Schichten auf dem Substrat angeordnet sein können;
abschnittsweises Belichten des Fotoresistfilms;
Tempern des belichteten Fotoresistfilms, wobei in den belichteten Abschnitten die säurelabilen Gruppen vom Polymer abgespalten werden;
Entwickeln des belichteten und getemperten Fotoresistfilms mit einer wässrig-alkalischen Entwicklerlösung, wobei die belichteten Abschnitte des Fotoresistfilms vom Substrat abgelöst werden und ein strukturierter Resist erhalten wird;
gegebenenfalls Ätzen der Schicht des elektrischen leitfähigen zweiten Polymers;
gegebenenfalls Freisetzen der Ankergruppen des ersten Polymers;
Aufbringen einer Lösung der biokompatiblen Verbindung, wobei die biokompatible Verbindung an die Ankergruppen des ersten Polymers koordiniert wird;
Entfernen überschüssiger Lösung der biokompatiblen Verbindung.

3. Verfahren nach Anspruch 1, wobei auf dem Substrat eine Schicht erzeugt wird, die das erste Polymer und den Fotosäurebildner umfasst, die Schicht strukturiert wird, so dass eine strukturierte Schicht erhalten wird, und auf die strukturierte Schicht eine Schicht des zweiten Polymers aufgebracht wird.

4. Verfahren nach Anspruch 3, wobei das zweite Polymer als Lösung auf die strukturierte Schicht aufgebracht wird.

5. Verfahren nach Anspruch 3 oder 4, wobei das zweite Polymer an das erste Polymer koordiniert wird.

6. Verfahren nach einem der vorhergehenden Ansprüche, wobei die Ankergruppen für die Anknüpfung der biokompatiblen Verbindung als Reaktivankergruppe ausgebildet sind, an welche die biokompatible Verbindung unter Ausbildung einer kovalenten Bindung koordiniert wird.

7. Verfahren nach Anspruch 6, wobei die Reaktivankergruppe ausgewählt ist aus der Gruppe, die gebildet ist aus Carbonsäureanhydrid, Epoxid, Isocyanat, Glycidylether, Amin, Alkylhalogenid, Thiol und Acylhalogenid.

8. Verfahren nach einem der vorhergehenden Ansprüche, wobei die biokompatible Verbindung eine Aminogruppe und/oder eine Hydroxylgruppe aufweist, über welche die biokompatible Verbindung an die Ankergruppe des ersten Polymers koordiniert wird.

9. Verfahren nach Anspruch 8, wobei die biokompatible Verbindung einen Spacer umfasst, welcher die Aminogruppe oder die Hydroxylgruppe für die Koordination der biokompatiblen Verbindung an die Ankergruppe des ersten Polymers trägt.

10. Verfahren nach einem der vorhergehenden Ansprüche, wobei die biokompatible Verbindung eine Aminosäure oder ein Peptid umfasst.

11. Verfahren nach einem der vorhergehenden Ansprüche, wobei die biokompatible Verbindung ein oligomeres oder polymeres Urethan ist.

12. Verfahren nach einem der vorhergehenden Ansprüche, wobei das Substrat ein Mikrochip ist.

13. Verfahren nach einem der vorhergehenden Ansprüche, wobei das elektrisch leitfähige Polymer ausgewählt ist aus der Gruppe, die gebildet ist aus elektrisch leitfähigem Polyanilin, elektrisch leitfähigem Polythiophen, elektrisch leitfähigem Polypyrrol und elektrisch leitfähigen Polyvinylphenylen.

14. Biokompatibler Mikrochip, umfassend:
ein Substrat, welches mikroelektronische Schaltungen umfasst, sowie eine auf dem Substrat angeordnete strukturierte Schicht, welche ein erstes Polymer enthält, das Ankergruppen aufweist, an die eine biokompatible Verbindung koordiniert ist, sowie ein zweites Polymer, das elektrisch leitfähig ist.

15. Biokompatibler Mikrochip nach Anspruch 14, wobei das erste Polymer und das zweite Polymer in getrennten Schichten angeordnet ist.

## Claims

1. Process for the production of biocompatible structures, a chemically amplified photoresist film which contains a first polymer which comprises anchor groups for linking a biocompatible compound and a second polymer which is electrically conductive being applied to a substrate and being structured so that a structured resist is obtained, and the structured resist being treated with a biocompatible compound so that the biocompatible compound is coordinated to the anchor groups of the first polymer.

2. Process according to Claim 1, comprising the steps:
application of a photoresist film to a substrate, the photoresist film containing the following components:
a first polymer which comprises acid-labile groups which, after their elimination, liberate a polar group with the result that the solubility of the first polymer in aqueous alkaline developers is increased, and which furthermore has anchor groups for linking a biocompatible compound, it also being possible for the anchor groups to be present in protected form;
a photo acid generator;
a second polymer which is electrically conductive, it being possible to arrange first and second polymer as a mixture or as successively arranged layers on the substrate;
section-by-section exposure of the photoresist film;
heating of the exposed photoresist film, the acid-labile groups being eliminated from the polymer in the exposed sections;
development of the exposed and heated photoresist film with an aqueous alkaline developer solution, the exposed sections of the photoresist film being detached from the substrate and a structured resist being obtained;
optionally etching of the layer of the electrically conductive second polymer;
optionally liberation of the anchor groups of the first polymer;
application of a solution of a biocompatible compound, the biocompatible compound being coordinated to the anchor groups of the first polymer;
removal of excess solution of the biocompatible compound.

3. Process according to Claim 1, a layer which comprises the first polymer and the photo acid generator being produced on the substrate, the layer being structured so that a structured layer is obtained, and a layer of the second polymer being applied to the structured layer.

4. Process according to Claim 3, the second polymer being applied as a solution to the structured layer.

5. Process according to Claim 3 or 4, the second polymer being coordinated to the first polymer.

6. Process according to any of the preceding claims, the anchor groups for linking the biocompatible compound being in the form of reactive anchor groups to which the biocompatible compound is coordinated with formation of a covalent bond.

7. Process according to Claim 6, the reactive anchor group being selected from the group consisting of carboxylic anhydride, epoxide, isocyanate, glycidyl ether, amine, alkyl halide, thiol and acyl halide.

8. Process according to any of the preceding claims, the biocompatible compound having an amino group and/or a hydroxyl group, via which the biocompatible compound is coordinated to the anchor group of the first polymer.

9. Process according to Claim 8, the biocompatible compound comprising a spacer which carries the amino group or the hydroxyl group for the coordination of the biocompatible compound to the anchor group of the first polymer.

10. Process according to any of the preceding claims, the biocompatible compound comprising an amino acid or a peptide.

11. Process according to any of the preceding claims, the biocompatible compound being an oligomeric or polymeric urethane.

12. Process according to any of the preceding claims, the substrate being a microchip.

13. Process according to any of the preceding claims, electrically conductive polymer being selected from the group consisting of electrically conductive polyaniline, electrically conductive polythiophene, electrically conductive polypyrrole and electrically conductive polyvinylphenylene.

14. Biocompatible microchip, comprising:
a substrate which comprises microelectronic circuits and a structured layer which is arranged on the substrate and contains a first polymer which has anchor groups to which a biocompatible compound is coordinated and a second polymer which is electrically conductive.

15. Biocompatible microchip according to Claim 14, the first polymer and the second polymer being arranged in separate layers.

## Revendications

1. Procédé de production de structures biocompatibles, dans lequel on dépose sur un substrat et on structure une pellicule de résist photosensible, amplifiée chimiquement, qui contient un premier polymère ayant des groupes d'ancrage pour la liaison d'un composé biocompatible et un deuxième polymère qui est conducteur de l'électricité, de manière à obtenir un résist structuré et on traite le résist structuré par un composé biocompatible, de manière à coordiner le composé biocompatible aux groupes d'ancrage du premier polymère.

2. Procédé suivant la revendication 1, comprenant les stades :
de dépôt d'une pellicule de résist photosensible sur un substrat, la pellicule de résist photosensible contenant les constituants suivants :
un premier polymère qui comprend des groupes labiles par un acide qui, après leur scission, libèrent un groupe polaire, grâce à quoi la solubilité du premier polymère dans des révélateurs aqueux-alcalins est augmentée et qui a, en outre, des groupes d'ancrage pour la liaison d'un composé biocompatible, les groupes d'ancrage pouvant se présenter aussi sous forme protégée ;
un générateur d'acide photosensible ;
un deuxième polymère qui est conducteur de l'électricité, le premier et le deuxième polymères pouvant être disposés sur le substrat sous la forme d'un mélange ou sous la forme de couches superposées ;
d'insolation par parties de la pellicule de résist photosensible ;
de recuit de la pellicule de résist photosensible insolée, les groupes labiles par des acides étant scindés du polymère dans les parties insolées ;
de développement de la pellicule de résist photosensible insolée et recuite par une solution de révélateur aqueuse alcaline, les parties insolées de la pellicule de résist photosensible étant séparées du substrat et un résist structuré étant obtenu ;
le cas échéant, d'attaque de la couche du deuxième polymère conducteur de l'électricité ;
le cas échéant, de libération des groupes d'ancrage du premier polymère ;
de dépôt d'une solution du composé biocompatible, le composé biocompatible étant coordiné sur les groupes d'ancrage du premier polymère ;
d'élimination de la solution en excès du composé biocompatible.

3. Procédé suivant la revendication 1, dans lequel on produit sur le substrat une couche qui comprend le premier polymère et le générateur d'acide photosensible, on structure la couche de manière à obtenir une couche structurée et l'on dépose une couche du deuxième polymère sur la couche structurée.

4. Procédé suivant la revendication 3, dans lequel on dépose le deuxième polymère sous la forme d'une solution sur la couche structurée.

5. Procédé suivant la revendication 3 ou 4, dans lequel on coordine le deuxième polymère sur le premier polymère.

6. Procédé suivant l'une des revendications précédentes, dans lequel les groupes d'ancrage pour la liaison du composé biocompatible sont constitués sous la forme de groupes d'ancrage réactifs sur lesquels le composé biocompatible est coordiné avec formation d'une liaison covalente.

7. Procédé suivant la revendication 6, dans lequel on choisit le groupe d'ancrage réactif dans le groupe qui est formé d'un anhydride d'un acide carboxylique, d'un époxyde, d'un isocyanate, d'un oxyde de glycidyle, d'une amine, d'un halogénure d'alcoyle, d'un thiol et d'un halogénure d'acyle.

8. Procédé suivant l'une des revendications précédentes, dans lequel le composé biocompatible comporte un groupe amino et/ou un groupe hydroxyle par lequel le composé biocompatible est coordiné sur le groupe d'ancrage du premier polymère.

9. Procédé suivant la revendication 8, dans lequel le composé biocompatible comprend un intercalaire qui porte le groupe amino ou le groupe hydroxyle pour la coordination du composé biocompatible au groupe d'ancrage du premier polymère.

10. Procédé suivant l'une des revendications précédentes, dans lequel le composé biocompatible comprend un acide aminé ou un peptide.

11. Procédé suivant l'une des revendications précédentes, dans lequel le composé biocompatible est un uréthane oligomère ou polymère.

12. Procédé suivant l'une des revendications précédentes, dans lequel le substrat est une micropuce.

13. Procédé suivant l'une des revendications précédentes, dans lequel on choisit le polymère conducteur de l'électricité dans le groupe qui est formé d'une polyaniline conductrice de l'électricité, d'un polythiophène conducteur de l'électricité, d'un polypyrrol conducteur de l'électricité et d'un polyvinylphénylène conducteur de l'électricité.

14. Micropuce biocompatible comprenant :
un substrat qui comprend des circuits microélectroniques, ainsi q'une couche structurée qui est disposée sur le substrat et qui contient un premier polymère ayant des groupes d'ancrage sur lesquels est coordiné un composé biocompatible, ainsi qu'un deuxième polymère qui est conducteur de l'électricité.

15. Micropuce biocompatible suivant la revendication 14, dans laquelle le premier polymère et le deuxième polymère sont disposés en des couches distinctes.
